# EUROPÄISCHE PATENTSCHRIFT

(11) **EP 4 498 106 B1**
(45) Veröffentlichungstag und Bekanntmachung des Hinweises auf die Patenterteilung: **12.11.2025**
(21) Anmeldenummer: 23188478.4
(22) Anmeldetag: 28.07.2023
(51) Int. Cl.: G01R 33/28, A61B 6/04, A61B 5/00, A61B 5/055, A61B 6/03

(54) **VORRICHTUNG UND VERFAHREN ZU EINEM ERFASSEN EINER POSITION EINES ZU UNTERSUCHENDEN BEREICHS**
DEVICE AND METHOD FOR DETECTING A POSITION OF AN AREA TO BE EXAMINED
DISPOSITIF ET PROCÉDÉ DE DÉTECTION D'UNE POSITION D'UNE ZONE À EXAMINER

(43) Veröffentlichungstag der Anmeldung: 29.01.2025
(73) Patentinhaber: Siemens Healthineers AG, 91301 Forchheim (DE)
(72) Erfinder: Sukkau, Johann, 91074 Herzogenaurach (DE)
(74) Vertreter: Siemens Healthineers Patent Attorneys

(56) Entgegenhaltungen:
- DE-A1- 102012 214 283
- DE-A1- 102021 202 978

## Beschreibung

Die vorliegende Erfindung betrifft eine medizinische Bildgebungsvorrichtung mit einem Liegentisch, auf dem ein Objekt für eine medizinische Bildgebungsuntersuchung positionierbar ist und der in einen Aufnahmebereich der medizinischen Bildgebungsvorrichtung einfahrbar ist, einer ersten Erfassungsvorrichtung, die zu einem Erfassen einer Position eines zu untersuchenden Bereich des Objekts ausgebildet ist, einer Projektionsvorrichtung, die zu einer Projektion und/oder Anzeige einer Feedback-Markierung an der erfassten Position des zu untersuchenden Bereich ausgebildet ist, und einer Linearachseneinheit, die eine Antriebseinheit zu einem Bewegen von zumindest der Markiervorrichtung umfasst, wobei die Linearachseneinheit parallel zur Längsrichtung des Liegentischs über dem Liegentisch angeordnet ist. Des Weiteren umfasst die Erfindung ein Verfahren zu einem Erfassen einer Position eines zu untersuchenden Bereichs eines Objekts mittels einer ersten Erfassungsvorrichtung für eine medizinische Bildgebungsuntersuchung.

Unabhängig vom grammatikalischen Geschlecht eines bestimmten Begriffes sind Personen mit männlicher, weiblicher oder anderer Geschlechteridentität mit umfasst.

Für eine medizinische Bildgebungsuntersuchung, insbesondere eine Magnetresonanzuntersuchung, ist es erforderlich, dass der Patient, insbesondere ein zu untersuchender Bereich des Patienten, korrekt im Isozentrum der medizinischen Bildgebungsvorrichtung positioniert wird. Beispielsweise wird dabei der zu untersuchende Bereich des Patienten mit einem Laserstrahl, der an einer Frontseite einer Magnetresonanzvorrichtung angeordnet ist, markiert. Hierzu muss ein Liegentisch, auf dem der Patient für die Magnetresonanzuntersuchung positioniert ist, solange in z-Richtung, die der Längsrichtung des Liegentischs entspricht, verfahren werden, bis der zu untersuchende Bereich des Patienten mit der Lasermarkierung übereinstimmt. Nach der Markierung des zu untersuchenden Bereichs wird anschließend automatisch der Liegentisch zusammen mit dem Patienten in einen Aufnahmebereich der Magnetresonanzvorrichtung gefahren, wobei hierbei der zu untersuchende Bereich bzw. der markierte Bereich des Patienten im Isozentrum positioniert wird. Jedoch ist eine derartige Positionierung für einen unerfahrenen und/oder ungeübten Benutzer sehr schwierig, so dass die Positionierung einen hohen Zeitaufwand benötigt und/oder es auch zu Fehlpositionierungen kommen kann.

Aus DE 10 2021 202 978 A1 ist eine Vorrichtung zur Erfassung und Markierung eines zu untersuchenden Bereichs eines Patienten bekannt, wobei die Vorrichtung an einer Decke über dem Liegentisch angeordnet ist. Hierbei wird der zu untersuchende Bereich von einem Benutzer mit einem Markierobjekt markiert und von einer Erfassungsvorrichtung erfasst. Anschließend erfolgt eine Projektion einer Feedback-Markierung an der erfassten Position mittels einer Projektionsvorrichtung. Wenn die Position der Feedback-Markierung mit der Position des Markierobjekts übereinstimmt, wird anhand dieser Position eine Verschiebung des Liegentischs bis zum Isozentrum ermittelt. Jedoch weist dieses Verfahren den Nachteil auf, dass die Projektionsvorrichtung erst nach Erfassung des Markierobjekts an die entsprechende Position nachgeführt wird. Dieses Nachführen kann einige Sekunden in Anspruch nehmen, was zu einer geringen Akzeptanz bei Benutzern führt.

Der vorliegenden Erfindung liegt insbesondere die Aufgabe zugrunde, eine einfache und schnelle Markierung des zu untersuchenden Bereichs zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer medizinischen Bildgebungsvorrichtung umfassend:
- einen Liegentisch, auf dem ein Objekt für eine medizinische Bildgebungsuntersuchung positionierbar ist und der in einen Aufnahmebereich der medizinischen Bildgebungsvorrichtung einfahrbar ist,
- eine erste Erfassungsvorrichtung, die zu einem Erfassen einer Position eines zu untersuchenden Bereichs des Objekts für eine medizinische Bildgebungsuntersuchung ausgebildet ist,
- eine Projektionsvorrichtung, die zu einer Projektion und/oder Anzeige einer Feedback-Markierung an der erfassten Position des zu untersuchenden Bereichs ausgebildet ist,
- einer Linearachseneinheit, die eine Antriebseinheit zu einem Bewegen der Projektionsvorrichtung umfasst, wobei die Linearachseneinheit parallel zur Längsrichtung des Liegentischs über dem Liegentisch angeordnet ist.

Erfindungsgemäß weist die medizinische Bildgebungsvorrichtung eine zweite Erfassungsvorrichtung auf, die zu einem Erfassen von Positionsdaten eine medizinischen Bedienpersonals während eines Vorbereiten des Objekts für die medizinische Bildgebungsuntersuchung ausgebildet ist, wobei die Projektionsvorrichtung anhand der erfassten Positionsdaten der zweiten Erfassungsvorrichtung an eine Position des medizinischen Bedienpersonals bezogen auf eine Längsrichtung des Liegentischs an der Linearachseneinheit während der Vorbereitung des Objekts nachgeführt wird.

Die medizinische Bildgebungsvorrichtung kann dabei eine Computertomografie-Vorrichtung (CT-Vorrichtung), eine PET-Vorrichtung (Positron-Emissions-Tomografie-Vorrichtung), eine Magnetresonanzvorrichtung oder eine Kombination von mehreren medizinischen Bildgebungsvorrichtung umfassen, wie beispielsweise eine Magnetresonanz-PET-Vorrichtung oder eine CT-PET-Vorrichtung umfassen.

Die medizinische Bildgebungsvorrichtung ist bevorzugt zu einem Erfassen von medizinischen und/oder diagnostischen Bilddaten eines Patienten und/oder Objekts ausgelegt und/oder ausgebildet.

Die medizinische Bildgebungsvorrichtung umfasst bevorzugt eine Scannereinheit zur Erfassung der medizinischen und/oder diagnostischen Bilddaten. Die Scannereinheit umgibt dabei einen Aufnahmebereich der medizinischen Bildgebungsvorrichtung. Der Aufnahmebereich ist bevorzugt zylinderförmig ausgebildet und zu einer Aufnahme des Patienten und/oder des Objekts, insbesondere des zu untersuchenden Bereichs des Patienten und/oder Objekts, für eine medizinische Bildgebungsuntersuchung ausgebildet.

Das zu untersuchende Objekt umfasst bevorzugt einen Patienten. Insbesondere soll mittels der medizinischen Bildgebungsuntersuchung eine klinische und/diagnostische Fragestellung am Patienten geklärt werden. Zudem kann das Objekt auch ein Phantom für beispielsweise Justage-Messungen und/oder weitere, dem Fachmann als sinnvoll erscheinende Objekte umfassen.

Für eine medizinische Bildgebungsuntersuchung wird das Objekt, insbesondere der Patient, insbesondere der zu untersuchende Bereich des Objekts, insbesondere des Patienten, innerhalb des Aufnahmebereich der medizinischen Bildgebungsvorrichtung positioniert. Innerhalb des Aufnahmebereich ist bevorzugt das Field of View (FOV) und/oder ein Isozentrum der medizinischen Bildgebungsvorrichtung angeordnet. Das FOV umfasst bevorzugt einen Erfassungsbereich der medizinischen Bildgebungsvorrichtung, innerhalb dessen die Bedingungen für eine Erfassung von medizinischen Bilddaten innerhalb des Aufnahmebereichs vorliegen, wie beispielsweise ein homogenes Grundmagnetfeld bei einer Ausbildung der medizinischen Bildgebungsvorrichtung als Magnetresonanzvorrichtung. Das Isozentrum der medizinischen Bildgebungsvorrichtung umfasst bevorzugt den Bereich und/oder Punkt innerhalb der Magnetresonanzvorrichtung, der die optimalen und/oder idealen Bedingungen für die Erfassung von medizinischen Bilddaten aufweist. Beispielsweise umfasst bei einer Ausbildung der medizinischen Bildgebungsvorrichtung als Magnetresonanzvorrichtung das Isozentrum den homogensten Magnetfeldbereich innerhalb der Magnetresonanzvorrichtung.

Zu einem Einfahren und/oder Positionieren des Objekts und/oder des Patienten, insbesondere des zu untersuchenden Bereichs des Objekts und/oder des Patienten, innerhalb des Aufnahmebereichs umfasst die medizinische Bildgebungsvorrichtung eine Patientenlagerungsvorrichtung. Die Patientenlagerungsvorrichtung weist dabei einen Liegentisch auf, wobei der Liegentisch relativ zur Scannereinheit bewegbar ausgebildet ist. Bevorzugt ist hierbei der Liegentisch in Längsrichtung des Liegentischs und/oder in Längsrichtung des Aufnahmebereichs innerhalb des Aufnahmebereichs bewegbar ausgebildet, um den Patienten in einer Untersuchungsposition innerhalb des Aufnahmebereichs zu positionieren.

Die erste Erfassungsvorrichtung ist dazu ausgebildet, eine Position des zu untersuchenden Bereichs des Objekts, insbesondere des Patienten, für eine anstehende medizinische Bildgebungsuntersuchung zu erfassen. Dabei kann die erste Erfassungsvorrichtung eine Kamera, beispielsweise eine 2D-Kamera oder eine 3D-Kamera umfassen. Zudem kann die erste Erfassungsvorrichtung auch mehr als eine Kamera umfassen, beispielsweise ein Kamera-Array. Umfasst die erste Erfassungsvorrichtung eine Kamera, kann diese beispielsweise an einer Decke eines Untersuchungsraums, in dem die medizinische Bildgebungsvorrichtung angeordnet ist, angeordnet sein. Insbesondere ist die erste Erfassungsvorrichtung in einem Frontbereich vor einer Frontseite der Scannereinheit angeordnet. Der Frontbereich befindet sich dabei an die Frontseite angrenzend vor der Scannereinheit. Zudem kann die Erfassungsvorrichtung weitere Einheiten und/oder Sensoren zur Erfassung des zu untersuchenden Bereichs umfassen, die beispielswiese am Liegentisch angeordnet sein können.

Zur Erfassung des zu untersuchenden Bereichs wird vom Benutzer bevorzugt ein Markierelement auf dem zu untersuchenden Bereich des Objekts und/oder des Patienten positioniert, wobei das Markierelement von der ersten Erfassungsvorrichtung als dieses erkannt und dessen Position erfasst wird. Das Markierelement kann beispielsweise einen Finger des medizinischen Bedienpersonals oder auch einen handgeführten Gegenstand, beispielsweise einen Markierstab, umfassen.

Die Projektionsvorrichtung umfasst bevorzugt einen Videoprojektor und/oder eine Laser-Markierungseinheit, die zu einer Projektion und/oder Anzeige einer Feedback-Markierung an der von der ersten Erfassungsvorrichtung erfassten Position des zu untersuchenden Bereichs ausgebildet ist. Mittels der Feedback-Markierung erhält der Benutzer ein Feedback, ob der zu untersuchende Bereich des Objekts und/oder Patienten korrekt erfasst wurde.

Die Linearachseneinheit umfasst bevorzugt eine Antriebseinheit und eine Schiene, wobei mittels der Antriebseinheit zumindest die Projektionsvorrichtung auf der Schiene automatisch positioniert und/oder verschoben wird. Die Linearachseneinheit ist bevorzugt zusammen mit der Projektionsvorrichtung an einer Decke des Untersuchungsraums angeordnet. Insbesondere ist die Linearachseneinheit derart an der Decke des Untersuchungsraums angeordnet, dass der Liegentisch, der während einer Vorbereitung in einem Frontbereich vor einer Frontseite der Scannereinheit angeordnet ist, sich in einem Projektionsfeld der Projektionsvorrichtung befindet. Insbesondere ist auch die Linearachseneinheit zusammen mit der Projektionsvorrichtung in diesem Frontbereich vor der Scannereinheit angeordnet. Die Linearachseneinheit kann zudem eine Recheneinheit umfassen, wobei die Recheneinheit anhand der erfassten Positionsdaten der ersten Erfassungsvorrichtung eine Position entlang der Schiene für die Projektionsvorrichtung bestimmt. Mittels der Antriebseinheit wird bevorzugt ein Antriebsmoment für eine Bewegung der Projektionsvorrichtung auf der Schiene generiert. Die Schiene ist hierbei bevorzugt parallel zur Längsrichtung des Liegentischs ausgerichtet.

Eine detaillierte Beschreibung der ersten Erfassungsvorrichtung, der Projektionsvorrichtung und der Linearachseneinheit ist in DE 10 2021 202 978 A1 offenbart, wobei hiermit auf DE 10 2021 202 978 A1 verwiesen wird.

Die zweite Erfassungsvorrichtung ist zu einem Erfassen von Positionsdaten des medizinischen Bedienpersonals während einer Vorbereitung des Objekts für die anstehende medizinische Bildgebungsuntersuchung ausgelegt. Die Vorbereitung des Objekts umfasst dabei ein Positionieren des Objekts, beispielsweise des Patienten, auf dem Liegentisch. Zudem kann das Vorbereiten des Objekts auch ein Anbringen von Zusatzeinheiten am Objekt umfassen, wie beispielsweise an Positionieren von Lagerungskissen am Objekt und/oder ein Positionieren von lokalen Magnetresonanzspulen, die um den zu untersuchenden Bereich des Objekts, insbesondere des Patienten, angeordnet werden, und/oder ein Anbringen einer Einheit zur Erfassung von physiologischen Signalen, beispielsweise einer EKG-Einheit usw. Bevorzugt erfolgt das Erfassen von Positionsdaten des medizinischen Bedienpersonals kontinuierlich während der gesamten Vorbereitung des Objekts, insbesondere des Patienten, bis die Vorbereitung des Objekts für die anstehende medizinische Bildgebungsuntersuchung abgeschlossen ist.

Bevorzugt wird anhand der erfassten Positionsdaten eine Positionsinformation des medizinischen Bedienpersonals ermittelt. Bevorzugt umfasst die Positionsinformation des medizinischen Bedienpersonal eine Positionsinformation bezogen auf die z-Richtung der Scannereinheit und/oder die Längsrichtung des Liegentischs.

Anhand der erfassten Positionsdaten, insbesondere anhand der ermittelten Positionsinformation des medizinischen Bedienpersonals, bezogen auf die Längsrichtung des Liegentischs wird eine Position für die Projektionsvorrichtung bestimmt. Anschließend wird die Projektionsvorrichtung an diese Position verschoben und/oder verfahren, insbesondere mittels der Linearachseneinheit. Insbesondere wird hierbei die Projektionsvorrichtung in Längsrichtung des Liegentischs, die parallel zur z-Richtung ist, mittels der Linearachseneinheit verschoben und/oder verfahren. Die Position, an dem die Projektionsvorrichtung verfahren und/oder nachgeführt wird, entspricht im Wesentlichen der Position, die von der zweiten Erfassungsvorrichtung als die Position des medizinischen Bedienpersonals erfasst wurde. Die Position der Projektionsvorrichtung, in die diese verschoben wird, kann dabei um bis zu ±10cm von der erfassten Position des medizinischen Bedienpersonals abweichen. Die Position der Projektionsvorrichtung, in die diese verschoben wird, kann dabei um bis zu ±20cm von der erfassten Position des medizinischen Bedienpersonals abweichen. Die Position der Projektionsvorrichtung, in die diese verschoben wird, kann dabei um bis zu ±30cm von der erfassten Position des medizinischen Bedienpersonals abweichen.

Zudem kann es auch sein, dass neben der Projektionsvorrichtung auch die erste Erfassungsvorrichtung anhand der erfassten Positionsdaten der zweiten Erfassungsvorrichtung an eine Position des medizinischen Bedienpersonals in z-Richtung an der Linearachseneinheit während der Vorbereitung des Objekts nachgeführt wird. Alternativ zu einer Nachführung der ersten Erfassungsvorrichtung kann diese auch beispielsweise bei einer Ausbildung als hochauflösende Kamera, insbesondere einer 3D-Kamera, fest an der Decke des Untersuchungsraums angeordnet sein.

Derart kann die Projektionsvorrichtung stets in Längsrichtung des Liegentischs in der Nähe zum medizinischen Bedienpersonal angeordnet sein, so dass bei einem Auflegen des Markierelements zu einem Festlegen und/oder Erfassen des zu untersuchenden Bereichs des Objekts, insbesondere des Patienten, die Projektionsvorrichtung nur einen kurzen Verfahrweg aufweist oder bereits an der Position des zu untersuchenden Bereiches in Längsrichtung des Liegentischs angeordnet ist. Hierdurch kann die Projektionsvorrichtung besonders schnell und zeitsparend in die Position, in der eine Feedback-Markierung projiziert werden soll, gebracht und/oder gefahren werden. Damit kann auch das medizinische Bedienpersonal besonders zeitnah die Feedback-Markierung erhalten. Dies ermöglicht insbesondere eine einfache und schnelle Markierung des zu untersuchenden Bereichs, so dass ein Workflow des medizinischen Bedienpersonals nicht durch lange Wartezeiten verzögert wird. Hierdurch kann auch ein insbesondere versehentliches Abbrechen der Erfassung des zu untersuchenden Bereichs durch ein Wegnehmen des Markierelements von dem zu untersuchenden Bereich durch das medizinische Bedienpersonal reduziert und/oder verhindert werden.

Zusätzlich kann anhand der kontinuierlichen erfassten Positionsdaten des medizinischen Bedienpersonals mittels der zweiten Erfassungsvorrichtung eine Auswahl getroffen werden, ob die erste Erfassungsvorrichtung für eine Detektion und/oder Erfassung eines Markierelements eingeschaltet werden muss oder nicht. Hierdurch können Ressourcen, wie beispielsweise eine CPU-Zeit und/oder ein Energieverbrauch, eingespart werden. Ein weiterer Vorteil ist, dass hierdurch die erste Erfassungsvorrichtung keine speziellen und/oder kostenintensiven EMV-Maßnahmen (Maßnahmen zur elektromagnetischen Verträglichkeit), wie beispielsweise ein spezielles Schirmgehäuse bei einer Ausbildung der medizinischen Bildgebungsvorrichtung als Magnetresonanzvorrichtung, benötigt, da sie in der kritischen Magnetresonanz-Messzeit ausgeschaltet wird.

In einer vorteilhaften Weiterbildung der erfindungsgemäßen medizinischen Bildgebungsvorrichtung kann es vorgesehen sein, die medizinische Bildgebungsvorrichtung als Magnetresonanzvorrichtung ausgebildet ist. Insbesondere bei Magnetresonanzuntersuchungen, die eine längere Zeit beanspruchen, ist eine exakte Positionierung besonders wichtig. Damit wird auch die Aufenthaltszeit eines Patienten innerhalb des Aufnahmebereich auf die Messzeit reduziert und Messwiederholungen aufgrund von Fehlpositionierungen können somit verhindert werden.

Die Magnetresonanzvorrichtung umfasst bevorzugt eine als Magneteinheit ausgebildete Scannereinheit zur Erfassung der medizinischen und/oder diagnostischen Bilddaten. Hierbei umfasst die Magneteinheit einen Grundmagneten, eine Gradientenspuleneinheit und eine Hochfrequenzantenneneinheit. Die Hochfrequenzantenneneinheit ist hierbei fest innerhalb der Magneteinheit angeordnet. Die Magneteinheit umgibt dabei einen Aufnahmebereich der Magnetresonanzvorrichtung. Der Aufnahmebereich ist bevorzugt zylinderförmig ausgebildet und zu einer Aufnahme des Patienten und/oder des Objekts, insbesondere des zu untersuchenden Bereichs des Patienten und/oder Objekts, für eine Magnetresonanzuntersuchung ausgebildet.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Das Gradienten-System ist zu einer Erzeugung von Magnetfeldgradienten, die für eine Ortskodierung während einer Bildgebung verwendet werden, ausgebildet. Die Hochfrequenzantenneneinheit ist zu einer Aussendung von Hochfrequenzpulsen und/oder Anregungspulsen zur Generierung von Magnetresonanzsignalen ausgebildet.

In einer vorteilhaften Weiterbildung der erfindungsgemäßen medizinischen Bildgebungsvorrichtung kann es vorgesehen sein, dass die zweite Erfassungsvorrichtung eine Sensoreinheit zur Erfassung der Positionsdaten des medizinischen Bedienpersonals umfasst, wobei die Sensoreinheit ein Sichtfeld aufweist, das einen Bereich neben dem Liegentisch umfasst. Die Sensoreinheit kann dabei eine Kamera und/oder eine Wärmebildkamera und/oder weitere, dem Fachmann als sinnvoll erscheinende Sensoreinheiten umfassen. Während einer Vorbereitung des Objekts, insbesondere eines Patienten, für die anstehende medizinischen Bildgebungsuntersuchung, befindet sich der Liegentisch im dem Frontbereich vor der Frontseite der Scannereinheit. Der Bereich neben dem Liegentisch umfasst bevorzugt einen Aufenthaltsbereich des medizinischen Bedienpersonals, in dem sich dieses für eine Vorbereitung des Objekts, insbesondere eines Patienten, für eine medizinischen Bildgebungsuntersuchung aufhält. Derart kann vorteilhaft eine einfache und direkte Erfassung der Positionsdaten des medizinischen Bedienpersonals während einer Vorbereitung des Objekts, insbesondere eines Patienten, für eine medizinischen Bildgebungsuntersuchung erreicht werden. Zudem kann eine von der Erfassung des zu untersuchenden Bereichs unabhängige Erfassung der Positionsdaten des medizinischen Bedienpersonals bereitgestellt werden.

In einer vorteilhaften Weiterbildung der erfindungsgemäßen medizinischen Bildgebungsvorrichtung kann es vorgesehen sein, dass die Sensoreinheit zumindest einen Wärmebildsensor umfasst. Bevorzugt umfasst die Sensoreinheit ein Wärmebildsensorarray mit zwei oder mehr Wärmebildsensoren. Die einzelnen Wärmebildsensoren umfassen bevorzugt Infrarotsensoren, die zu einer Erfassung von Wärmestrahlung, insbesondere Infrarotstrahlung, ausgebildet sind. Durch die Verwendung von Wärmebildsensoren kann eine zuverlässige und robuste Erkennung des medizinischen Bedienpersonals zur Erfassung der Positionsdaten des medizinischen Bedienpersonals bereitgestellt werden. Zudem kann die Sensoreinheit zur Erfassung der Positionsdaten des medizinischen Bedienpersonals besonders günstig ausgeführt werden. Eine Erfassung der Positionsdaten des medizinischen Bedienpersonals mit einer Kamera, beispielsweise der Kamera der ersten Erfassungsvorrichtung, benötigt eine aufwendige Bildrekonstruktion und Analyse zur Erkennung des medizinischen Bedienpersonals, was mittels der Wärmebildsensoren deutlich einfacher ist. Insbesondere kann derart Energie und/oder Rechenleistung und/oder Erfassungszeit eingespart werden.

In einer vorteilhaften Weiterbildung der erfindungsgemäßen medizinischen Bildgebungsvorrichtung kann es vorgesehen sein, dass die zweite Erfassungsvorrichtung eine Sichtfeldmanipulationseinheit umfasst, das dazu ausgebildet ist, einen Bereich des Liegentischs aus dem Sichtfeld der Sensoreinheit auszublenden. Die Sichtfeldmanipulationseinheit kann dabei eine Blende und/oder ein Spiegelsystem mit zumindest einen Spiegel umfassen, die das Sichtfeld der Sensoreinheit derart umleiten und/oder einschränken, so dass der Bereich des Liegentischs nicht vom Sichtfeld der Sensoreinheit umfasst ist. Derart kann auf einfache Art und Weise eine Wärmestrahlung eines auf dem Liegentisch befindlichen Patienten während der Erfassung der Positionsdaten des medizinischen Bedienpersonals ausgeblendet werden. Insbesondere kann derart eine eindeutige und direkte Zuordnung einer erfassten Wärmestrahlung zu dem medizinischen Bedienpersonal bei einer Auswertung der mittels der zweiten Erfassungsvorrichtung erfassten Positionsdaten erfolgen und Fehler bei der Auswertung reduziert und/oder eliminiert werden.

In einer vorteilhaften Weiterbildung der erfindungsgemäßen medizinischen Bildgebungsvorrichtung kann es vorgesehen sein, dass die Sichtfeldmanipulationseinheit zu einer Teilung und/oder Umlenkung des Sichtfelds der Sensoreinheit in zwei Teilsichtfelder ausgebildet ist. Vorzugsweise deckt jedes Teilsichtfeld einen Bereich neben dem Liegentisch ab, wobei die beiden Teilsichtfelder an unterschiedlichen Bereichen neben dem Liegentisch angeordnet sind. Dabei kann ein erstes Teilsichtfeld den Bereich rechts des Liegentisch und ein zweites Teilsichtfeld den Bereich links des Liegentischs abdecken, wobei ein mittlerer Bereich, insbesondere der Bereich des Liegentischs, vom Sichtfeld der Sensoreinheit ausgeschlossen ist. Derart kann konstruktiv einfach ein auf dem Liegentisch positionierter Patient aus dem Sichtfeld der Sensoreinheit eliminiert werden. Zudem kann vorteilhaft jedes der beiden Teilsichtfelder genügend groß ausgebildet sein, um das medizinische Bedienpersonal zu lokalisieren. Insbesondere können derart mittels einer einzigen Sensoreinheit beide Bereiche neben dem Liegentisch für eine Lokalisierung des medizinischen Bedienpersonals erfasst werden.

In einer vorteilhaften Weiterbildung der erfindungsgemäßen medizinischen Bildgebungsvorrichtung kann es vorgesehen sein, dass die Sichtfeldmanipulationseinheit ein Spiegelsystem mit zwei Erfassungspfaden umfasst, wobei jeder Erfassungspfad zumindest einem Spiegel zu einer Umleitung eines Teilsichtfelds aufweist. Vorzugsweise wird dabei jeweils ein Teilsichtfeld von der Sensoreinheit auf den zumindest einen Spiegel und von dem zumindest einen Spiegel auf den Bereich neben dem Liegentisch gelenkt. Besonders vorteilhaft weist das Spiegelsystem zwei Spiegel für jeden Erfassungspfad auf, wobei jeweils ein Teilsichtfeld von der Sensoreinheit auf einen ersten Spiegel, von dem ersten Spiegel auf einen zweiten Spiegel und von dem zweiten Spiegel auf den Bereich neben dem Liegentisch gelenkt wird. Derart kann besonders einfach der Bereich des Liegentischs aus dem Sichtfeld der Sensoreinheit ausgeschlossen werden. Ein weiterer Vorteil dieser Ausgestaltung ist, dass eine Erfassung der zwei Teilsichtfelder mittels des Spiegelsystems deutlich günstiger ist als die zweite Erfassungsvorrichtung mit einem zweiten Wärmebildsensor auszustatten zur Aufteilung des Sichtfelds in zwei Teilsichtfelder.

In einer vorteilhaften Weiterbildung der erfindungsgemäßen medizinischen Bildgebungsvorrichtung kann es vorgesehen sein, dass die zweite Erfassungsvorrichtung eine Auswerteeinheit aufweist, die zu einer Auswertung der erfassten Positionsdaten ausgebildet ist. Die Auswerteeinheit umfasst bevorzugt einen Mikrokontroller. Zudem umfasst die Auswerteeinheit eine Software, die zu einer Auswertung der mittels der Sensoreinheit erfassten Positionsdaten, insbesondere des zumindest einen Wärmebildsensors, ausgebildet ist. Bevorzugt wird bei der Auswertung der mittels der Sensoreinheit erfassten Positionsdaten eine Positionsinformation des medizinischen Bedienpersonals in z-Richtung und/oder bezogen auf die Längsrichtung des Liegentischs von der Auswerteeinheit, insbesondere dem Mikrokontroller, ermittelt und/oder bestimmt. Derart kann eine direkte und einfache Auswertung für eine Lokalisierung und/oder eine Bestimmung der Positionsinformation des medizinischen Bedienpersonals bereitgestellt werden. Insbesondere kann derart die Auswertung der mittels der Sensoreinheit erfassten Positionsdaten unabhängig von einem Bestimmen und/oder Festlegen des zu untersuchenden Bereichs erfolgen.

In einer vorteilhaften Weiterbildung der erfindungsgemäßen medizinischen Bildgebungsvorrichtung kann es vorgesehen sein, dass die zweite Erfassungsvorrichtung ein Schirmgehäuse umfasst. Bevorzugt umfasst das Schirmgehäuse ein EMV(Elektromagnetische Verträglichkeit)-sicheres Schirmgehäuse, das eine elektromagnetische Strahlung von der zweiten Erfassungsvorrichtung abschirmt und/oder eine elektromagnetische Strahlung der zweiten Erfassungsvorrichtung von der Scannereinheit, insbesondere einer Magneteinheit einer Magnetresonanzvorrichtung, abschirmt. Derart kann eine unerwünschte Interaktion zwischen der zweiten Erfassungsvorrichtung und der Scannereinheit, insbesondere der Magneteinheit, vorteilhaft verhindert werden. Insbesondere kann ein störungsfreier Betrieb der zweiten Erfassungsvorrichtung und der Scannereinheit, insbesondere der Magneteinheit, gewährleistet werden.

In einer vorteilhaften Weiterbildung der erfindungsgemäßen medizinischen Bildgebungsvorrichtung kann es vorgesehen sein, dass die zweite Erfassungsvorrichtung eine optische Datenübertragungseinheit zu einem Übertragen einer Positionsinformation des medizinischen Bedienpersonals an die medizinische Bildgebungsvorrichtung und/oder an die Linearachseneinheit. Die optische Datenübertragungseinheit weist bevorzugt zumindest einen Lichtwellenleiter zur Datenübertragung auf. Zudem weist hierbei die zweite Erfassungsvorrichtung, insbesondere die Auswerteeinheit der zweiten Erfassungsvorrichtung, eine entsprechende optische Datenschnittstelle auf. Durch die Datenübertragung mittels der optischen Datenübertragungseinheit, insbesondere des zumindest einen Lichtwellenleiters, können unerwünschte Störungen der medizinischen Bildgebung, beispielsweise unerwünschte Artefakte in den medizinischen Bilddaten, verhindert werden.

Des Weiteren geht die Erfindung aus von einem Verfahren zu einem Erfassen einer Position eines zu untersuchenden Bereichs eines Objekts mittels einer ersten Erfassungsvorrichtung für eine medizinische Bildgebungsuntersuchung, umfassend die folgenden Verfahrensschritte:
- Vorbereiten des Objekts auf einem in einem Aufnahmebereich einer medizinischen Bildgebungsvorrichtung einfahrbaren Liegentisch, wobei das Vorbereiten des Objekts manuell durch ein medizinisches Bedienpersonal erfolgt,
- Erfassen von Positionsdaten des medizinischen Bedienpersonals mittels einer zweiten Erfassungsvorrichtung während des Positionierens des Objekts,
- Nachführen einer Projektionsvorrichtung in Längsrichtung des Liegentischs an eine Position des medizinischen Bedienpersonals, wobei das Nachführen auf Basis einer anhand der erfassten Positionsdaten ermittelten Positionsinformation des medizinischen Bedienpersonals erfolgt,
- Wiederholen der Verfahrensschritte des Positionieren des Objekts, des Erfassens der Positionsdaten des medizinischen Bedienpersonals und des Verfahrensschritts des Nachführens der Projektionsvorrichtung so lange, bis der zu untersuchende Bereich des Objekts mittels eines manuellen Positionierens eines Markierelements festgelegt wird,
- Erfassen der Position des Markierelements mittels der ersten Erfassungsvorrichtung, und
- Projektion und/oder Anzeige einer Feedback-Markierung durch eine Projektionsvorrichtung an der erfassten Position des zu untersuchenden Bereichs des Objekts.

Eine detaillierte Beschreibung eines Erfassens der Position des Markierelements und einer Projektion und/oder Anzeige einer Feedback-Markierung ist in DE 10 2021 202 978 A1 offenbart, wobei hiermit auf DE 10 2021 202 978 A1 verwiesen wird.

Das Nachführen der Projektionsvorrichtung erfolgt dabei mittels der Linearachseneinheit der medizinischen Bildgebungsvorrichtung.

Die Projektion und/oder Anzeige einer Feedback-Markierung umfasst dabei auch ein Positionieren der Projektionsvorrichtung an der Position des Markierelements bezogen auf die Längsrichtung des Liegentischs.

Derart kann die Projektionsvorrichtung stets in Längsrichtung des Liegentischs in der Nähe zum medizinischen Bedienpersonal angeordnet sein, so dass bei einem Auflegen des Markierelements zu einem Festlegen und/oder Erfassen des zu untersuchenden Bereichs des Objekts, insbesondere des Patienten, die Projektionsvorrichtung nur einen kurzen Verfahrweg aufweist oder bereits an der Position des zu untersuchenden Bereiches in Längsrichtung des Liegentischs angeordnet ist. Hierdurch kann die Projektionsvorrichtung besonders schnell und zeitsparend in die Position, in der eine Feedback-Markierung projiziert werden soll, gebracht und/oder gefahren werden. Damit kann auch das medizinische Bedienpersonal besonders zeitnah die Feedback-Markierung erhalten. Dies ermöglicht insbesondere eine einfache und schnelle Markierung des zu untersuchenden Bereichs, so dass ein Workflow des medizinischen Bedienpersonals nicht durch lange Wartezeiten verzögert wird. Hierdurch kann auch ein insbesondere versehentliches Abbrechen der Erfassung des zu untersuchenden Bereichs durch ein Wegnehmen des Markierelements von dem zu untersuchenden Bereich durch das medizinische Bedienpersonal reduziert und/oder verhindert werden.

Zusätzlich kann anhand der kontinuierlichen erfassten Positionsdaten des medizinischen Bedienpersonals mittels der zweiten Erfassungsvorrichtung eine Auswahl getroffen werden, ob die erste Erfassungsvorrichtung für eine Detektion und/oder Erfassung eines Markierelements eingeschaltet werden muss oder nicht. Hierdurch können Ressourcen, wie beispielsweise eine CPU-Zeit und/oder ein Energieverbrauch, eingespart werden. Ein weiterer Vorteil ist, dass hierdurch die erste Erfassungsvorrichtung keine speziellen und/oder kostenintensiven EMV-Maßnahmen (Maßnahmen zur elektromagnetischen Verträglichkeit), wie beispielsweise ein spezielles Schirmgehäuse bei einer Ausbildung der medizinischen Bildgebungsvorrichtung als Magnetresonanzvorrichtung, benötigt, da sie in der kritischen Magnetresonanz-Messzeit ausgeschaltet wird.

Die Vorteile des erfindungsgemäßen Verfahrens zu einem Erfassen eine Position eines zu untersuchenden Bereichs eines Objekts mittels einer ersten Erfassungsvorrichtung für eine medizinische Bildgebungsvorrichtung entsprechen im Wesentlichen den Vorteilen der erfindungsgemäßen medizinischen Bildgebungsvorrichtung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Verfahrens kann es vorgesehen sein, dass zum Erfassen der Positionsdaten des medizinischen Bedienpersonals in dem Sichtfeld der zweiten Erfassungsvorrichtung ein Bereich des Liegentischs ausgeblendet wird. Derart kann auf einfache Art und Weise eine Wärmestrahlung eines auf dem Liegentisch befindlichen Patienten während der Erfassung der Positionsdaten des medizinischen Bedienpersonals ausgeblendet werden. Umfasst die zweite Erfassungsvorrichtung einen Wärmebildsensor kann zudem eine eindeutige und direkte Zuordnung einer erfassten Wärmestrahlung zu dem medizinischen Bedienpersonal bei einer Auswertung der mittels der zweiten Erfassungsvorrichtung erfassten Positionsdaten erfolgen und Fehler bei der Auswertung reduziert und/oder eliminiert werden.

In einer vorteilhaften Weiterbildung des erfindungsgemäßen Verfahrens kann es vorgesehen sein, dass zum Erfassen der Positionsdaten des medizinischen Bedienpersonals ein Sichtfeld der zweiten Erfassungsvorrichtung geteilt und/oder umgelenkt wird. Die zweite Erfassungsvorrichtung weist hierzu ein Sichtfeldmanipulationseinheit auf, die das Sichtfeld teilt und/oder umlenkt. Bevorzugt weist die zweite Erfassungsvorrichtung, insbesondere die Sichtfeldmanipulationseinheit, ein Spiegelsystem auf, das zu einer Teilung und/oder Umlenkung des Sichtfelds in zwei Teilsichtfelder ausgebildet ist. Vorzugsweise deckt jedes Teilsichtfeld einen Bereich neben dem Liegentisch ab. Dabei kann ein erstes Teilsichtfeld den Bereich rechts des Liegentisch und ein zweites Teilsichtfeld den Bereich links des Liegentischs abdecken, wobei ein mittlerer Bereich, insbesondere der Bereich des Liegentischs, vom Sichtfeld der Sensoreinheit ausgeschlossen ist. Derart kann konstruktiv einfach ein auf dem Liegentisch positionierter Patient aus dem Sichtfeld der Sensoreinheit ausgeblendet und/oder eliminiert werden. Zudem kann vorteilhaft jedes der beiden Teilsichtfelder genügend groß ausgebildet sein, um das medizinische Bedienpersonal zu lokalisieren. Insbesondere können derart mittels einer einzigen Sensoreinheit beide Bereiche neben dem Liegentisch für eine Lokalisierung und Erfassung der Positionsdaten des medizinischen Bedienpersonals erfasst werden.

In einer vorteilhaften Weiterbildung des erfindungsgemäßen Verfahrens kann es vorgesehen sein, dass mittels der zweiten Erfassungsvorrichtung die erfassten Positionsdaten ausgewertet werden und eine ermittelte Positionsinformation des medizinischen Bedienpersonals mittels einer optischen Datenübertragungseinheit an eine Linearachseneinheit zur Positionierung der Projektionsvorrichtung übertragen werden. Bevorzugt weist hierbei die zweite Erfassungsvorrichtung eine Auswerteeinheit auf, wie beispielsweise einen Mikrokontroller. Die Auswerteeinheit, insbesondere der Mikrokontroller, kann dabei eine entsprechende optische Datenschnittstelle für eine Datenübertragung umfassen. Die optische Datenübertragungseinheit umfasst bevorzugt zumindest einen Lichtwellenleiter zur Datenübertragung an die Linearachseneinheit und/oder die medizinische Bildgebungsvorrichtung. Derart kann eine direkte Auswertung der erfassten Positionsdaten erfolgen. Zudem können unerwünschte Störungen der medizinische Bildgebung, beispielsweise unerwünschte Artefakte in den medizinische Bildgebungsdaten, durch die Datenübertragung mittels Lichtwellenleiter verhindert werden.

In einer vorteilhaften Weiterbildung des erfindungsgemäßen Verfahrens kann es vorgesehen sein, dass anhand der erfassten Position des zu untersuchenden Bereichs des Objekts eine Untersuchungsposition des Liegentischs berechnet wird. Dabei erfolgt das Berechnen der Untersuchungsposition des Liegentisch mittels einer Recheneinheit und/oder Steuereinheit der medizinischen Bildgebungsvorrichtung. Die Untersuchungsposition umfasst dabei diejenige Position des Liegentischs bezüglich der Scannereinheit, dass der zu untersuchende Bereich des Objekts, insbesondere des Patienten, innerhalb des Isozentrums der medizinischen Bildgebungsvorrichtung angeordnet ist. Derart kann eine einfache und zeitsparende Positionierung des zu untersuchenden Bereichs im Isozentrum der medizinischen Bildgebungsvorrichtung ermöglicht werden. Insbesondere kann derart das medizinische Bedienpersonal vorteilhaft bei der Positionierung eines Patienten innerhalb der medizinischen Bildgebungsvorrichtung unterstützt werden und ein Positionierungsworkflow für unerfahrene Benutzer vereinfacht werden.

In einer vorteilhaften Weiterbildung des erfindungsgemäßen Verfahrens kann es vorgesehen sein, dass der Liegentisch automatisch in die Untersuchungsposition gefahren wird für eine Erfassung von medizinische Bildgebungsdaten des zu untersuchenden Bereichs des Objekts. Hierbei kann ebenfalls eine einfache und zeitsparende Positionierung des zu untersuchenden Bereichs im Isozentrum der medizinischen Bildgebungsvorrichtung ermöglicht werden. Vorzugsweise weist hierzu die Patientenlagerungsvorrichtung eine entsprechende Horizontaleinstelleinheit und eine Positionssteuereinheit auf zu Positionierung des Liegentischs in der Untersuchungsposition.

Des Weiteren geht die Erfindung aus von einem Computerprogrammprodukt, welches ein Programm umfasst und direkt in einem Speicher einer programmierbaren Steuerungseinheit ladbar ist, mit Programmmitteln, um ein Verfahren zu einem Erfassen einer Position eines zu untersuchenden Bereichs eines Objekts mittels einer ersten Erfassungsvorrichtung für eine medizinische Bildgebungsuntersuchung auszuführen, wenn das Programm in der Steuerungseinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit der medizinischen Bildgebungsvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße als Magnetresonanzvorrichtung ausgebildete medizinische Bildgebungsvorrichtung in einer schematischen Darstellung,
- Fig. 2: eine zweite Erfassungsvorrichtung der Magnetresonanzvorrichtung und
- Fig. 3: ein erfindungsgemäßes Verfahren zu einem Erfassen einer Position eines zu untersuchenden Bereichs eines Objekts mittels einer ersten Erfassungsvorrichtung für eine medizinische Bildgebungsuntersuchung.

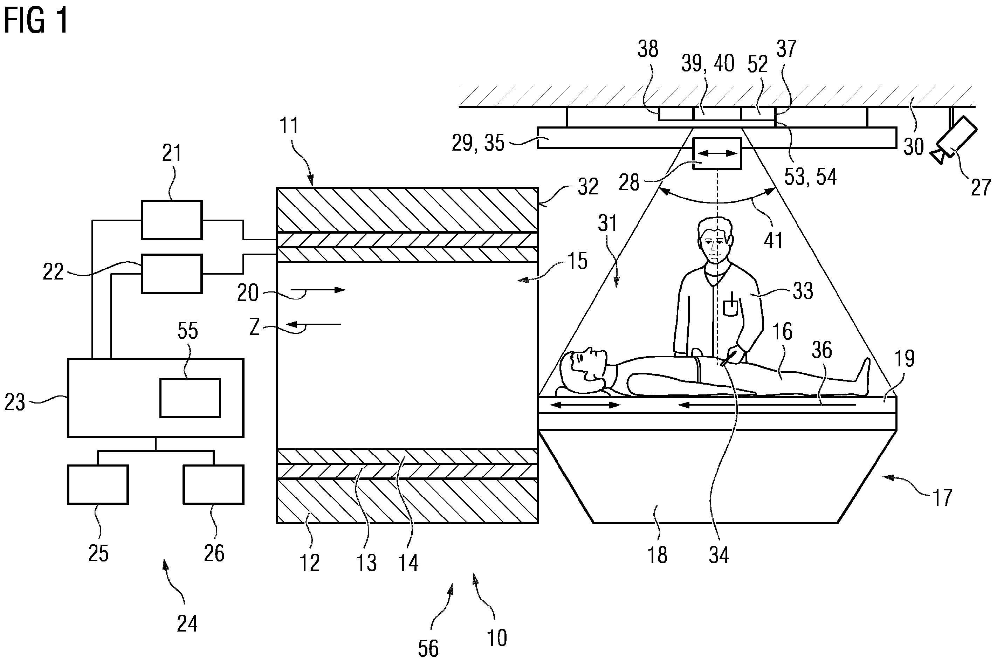

In Fig. 1 ist eine medizinische Bildgebungsvorrichtung 56 schematisch dargestellt. Die medizinische Bildgebungsvorrichtung 56 ist im vorliegenden Ausführungsbeispiel von einer Magnetresonanzvorrichtung 11 gebildet, wobei beispielhaft die vorliegende Erfindung anhand der Magnetresonanzvorrichtung erläutert wird. Die vorliegende Erfindung ist jedoch nicht auf Ausgestaltung der medizinischen Bildgebungsvorrichtung 56 auf eine Magnetresonanzvorrichtung 11 beschränkt und weitere Ausgestaltungen der medizinischen Bildgebungsvorrichtung 56 sind jederzeit denkbar.

Die Magnetresonanzvorrichtung 10 umfasst eine als Magneteinheit 11 ausgebildete Scannereinheit mit einem Grundmagneten 12, einer Gradientenspuleneinheit 13 und einer Hochfrequenzantenneneinheit 14. Zudem weist die medizinische Bildgebungsvorrichtung 56, insbesondere die Magnetresonanzvorrichtung 10, einen Aufnahmebereich 15 auf zu einer Aufnahme eines Objekts und/oder Patienten 16 für eine Magnetresonanzuntersuchung. Der Aufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Aufnahmebereich 15 jederzeit denkbar.

Für eine Positionierung des Objekts und/oder Patienten 16, insbesondere eines zu untersuchenden Bereichs des Patienten 16, innerhalb des Aufnahmebereich 15 weist die medizinische Bildgebungsvorrichtung 56, insbesondere die Magnetresonanzvorrichtung 10, eine Patientenlagerungsvorrichtung 17 auf. Die Patientenlagerungsvorrichtung 17 weist eine Basiseinheit 18 und einen bezüglich der Basiseinheit 18 bewegbaren Liegentisch 19 auf. Der Liegentisch 19 ist für eine Positionierung des Objekts und/oder Patienten 16, insbesondere des zu untersuchenden Bereichs des Patienten 16, bewegbar innerhalb des Aufnahmebereich 15 ausgebildet. Insbesondere ist hierbei der Liegentisch 19 in Richtung einer Längserstreckung des Aufnahmebereich 15 und/oder in z-Richtung bewegbar gelagert.

Der Grundmagnet 12 der Magneteinheit 11 ist zu einem Erzeugen eines starken und insbesondere konstanten Grundmagnetfelds 20 ausgebildet. Dabei kann der Grundmagnet 12 beispielsweise als supraleitender Grundmagnet 12 oder auch als Dauermagnet ausgebildet sein. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 21 der Magnetresonanzvorrichtung 10 gesteuert. Die Hochfrequenzantenneneinheit 14 der Magneteinheit 11 ist zu einer Anregung einer Polarisation, die sich in dem von dem Grundmagneten 12 erzeugten Grundmagnetfeld 20 einstellt, ausgebildet. Die Hochfrequenzantenneneinheit 14 wird von einer Hochfrequenzantennensteuereinheit 22 der Magnetresonanzvorrichtung 10 gesteuert und strahlt hochfrequente Magnetresonanzsequenzen in den Aufnahmebereich 15 der Magnetresonanzvorrichtung 10 ein.

Zu einer Steuerung des Grundmagneten 12, der Gradientensteuereinheit 21 und zur Steuerung der Hochfrequenzantennensteuereinheit 22 weist die Magnetresonanzvorrichtung 10 eine Systemsteuereinheit 23 auf. Die Systemsteuereinheit 23 steuert zentral die Magnetresonanzvorrichtung 10, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die medizinische Bildgebungsvorrichtung 56, insbesondere die Magnetresonanzvorrichtung 10, eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Darstellungseinheit 25, beispielsweise auf zumindest einem Monitor, der Benutzerschnittstelle 24 für ein medizinisches Bedienpersonal 33 angezeigt werden. Weiterhin weist die Benutzerschnittstelle 24 eine Eingabeeinheit 26 auf, mittels der Informationen und/oder Parameter während eines Messvorgangs von einem medizinischen Bedienpersonal 33 eingegeben werden können.

Zu einer Positionierung des Patienten 16 weist die medizinische Bildgebungsvorrichtung 56, insbesondere die Magnetresonanzvorrichtung 10, eine erste Erfassungsvorrichtung 27, eine Projektionsvorrichtung 28 und eine Linearachseneinheit 29 auf. Die erste Erfassungsvorrichtung 27 ist zu einem Erfassen einer Position eines zu untersuchenden Bereichs des Objekts und/oder des Patienten 16 für eine Magnetresonanzuntersuchung ausgebildet. Hierzu weist die erste Erfassungsvorrichtung 27 bevorzugt eine Kamera, insbesondere eine 2D-Kamera und/oder eine 3D-Kamera, auf. Die erste Erfassungsvorrichtung 27 ist im vorliegenden Ausführungsbeispiel an einer Decke 30 des Untersuchungsraums, in dem die Magneteinheit 11 angeordnet ist, angeordnet. Dabei ist die erste Erfassungsvorrichtung 27 in einem Frontbereich 31 vor einer Frontseite 32 der Magneteinheit 11 angeordnet, um den Patienten 16 und/oder den Liegentisch 19 vor einem Einfahren in den Aufnahmebereich 15 zu erfassen.

Zur Erfassung des zu untersuchenden Bereichs wird vom medizinischen Bedienpersonal 33 bevorzugt ein Markierelement 34 an dem zu untersuchenden Bereich positioniert, das von der ersten Erfassungsvorrichtung 27 erkannt und erfasst wird. Das Markierelement 34 kann beispielsweise einen Finger des medizinischen Bedienpersonals 33 oder auch einen handgeführten Gegenstand, beispielsweise einen Markierstab, umfassen.

Die Projektionsvorrichtung 28 umfasst bevorzugt einen Videoprojektor und/oder eine Laser-Markierungseinheit, die zu einer Projektion und/oder Anzeige einer Feedback-Markierung an der von der ersten Erfassungsvorrichtung 27 erfassten Position ausgebildet ist. Die Feedback-Markierung ist für den Benutzer, insbesondere das medizinische Bedienpersonal 33, vorgesehen, um eine die von der ersten Erfassungsvorrichtung 27 erfasste Position des zu untersuchenden Bereichs zu kontrollieren.

Die Linearachseneinheit 29 ist bevorzugt an der Decke 30 des Untersuchungsraums angeordnet. Insbesondere ist die Linearachseneinheit 29 derart an der Decke 30 des Untersuchungsraums angeordnet, dass der Liegentisch 19, der während einer Vorbereitung in dem Frontbereich 31 vor der Frontseite 32 der Magneteinheit 11 angeordnet ist, sich in einem Projektionsfeld der Projektionsvorrichtung 28 befindet. Insbesondere ist auch die Linearachseneinheit 29 zusammen mit der Projektionsvorrichtung 28 in dem Frontbereich 31 vor der Frontseite 32 der Magneteinheit 11 angeordnet. Die Linearachseneinheit 29 umfasst bevorzugt eine nicht näher dargestellte Antriebseinheit und eine Schiene 35, wobei mittels der Antriebseinheit die Projektionsvorrichtung 28 auf der Schiene 35 automatisch positioniert und/oder verschoben wird. Hierbei kann die Linearachseneinheit 29 zudem eine Recheneinheit umfassen, die anhand der erfassten Positionsdaten der ersten Erfassungsvorrichtung 27 eine Position entlang der Schiene 35 für die Projektionsvorrichtung 28 für eine Projektion der Feedback-Markierung bestimmt. Die Schiene 35 ist hierbei bevorzugt parallel zur Längsrichtung 36 des Liegentischs 19 ausgerichtet.

Die medizinische Bildgebungsvorrichtung 56, insbesondere die Magnetresonanzvorrichtung 10, weist des Weiteren eine zweite Erfassungsvorrichtung 37 auf, die zu einer Erfassung von Positionsdaten des medizinischen Bedienpersonals 33 während eines Vorbereitens des Objekts, insbesondere des Patienten 16, für die Magnetresonanzuntersuchung ausgebildet ist. Die erfassten Positionsdaten werden dazu verwendet, die Projektionsvorrichtung 28 an der Linearachseneinheit 29 an eine Position des medizinischen Bedienpersonals 33 in Längsrichtung 36 des Liegentischs 19 während der Vorbereitung des Objekts und/oder des Patienten 16 nachzuführen. Anhand der erfassten Positionsdaten, insbesondere anhand der ermittelten Position in Längsrichtung 36 des Liegentischs 19, wird eine Position für die Projektionsvorrichtung 28 bestimmt und die Projektionsvorrichtung 28 an diese Position verschoben und/oder verfahren, insbesondere mittels der Linearachseneinheit 29. Die Position, an die die Projektionsvorrichtung 28 verfahren und/oder nachgeführt wird, entspricht im Wesentlichen der Position, die von der zweiten Erfassungsvorrichtung 37 als die Position des medizinischen Bedienpersonals 33 erfasst wurde. Die Position der Projektionsvorrichtung 28, in die diese verschoben wird, kann dabei um bis zu ±10cm von der erfassten Position des medizinischen Bedienpersonals 33 abweichen. Die Position der Projektionsvorrichtung 28, in die diese verschoben wird, kann dabei um bis zu ±20cm von der erfassten Position des medizinischen Bedienpersonals 33 abweichen. Die Position der Projektionsvorrichtung 28, in die diese verschoben wird, kann dabei um bis zu ±30cm von der erfassten Position des medizinischen Bedienpersonals 33 abweichen.

Die zweite Erfassungsvorrichtung 37 ist der Decke 30 des Untersuchungsraums angeordnet. Zudem weist die zweite Erfassungsvorrichtung 37 ein Schirmgehäuse 38 auf, innerhalb dessen eine Sensoreinheit 39 zur Erfassung der Positionsdaten des medizinischen Bedienpersonals 33 angeordnet ist. Das Schirmgehäuse 38 umfasst bevorzugt ein EMV-dichtes Schirmgehäuse, das eine elektromagnetische Strahlung von der zweiten Erfassungsvorrichtung 37 abschirmt.

Zur Erfassung der Position des medizinischen Bedienpersonals 33 weist die zweite Erfassungsvorrichtung 37 die Sensoreinheit 39 auf. Im vorliegenden Ausführungsbeispiel weist die Sensoreinheit 39 zumindest einen Wärmebildsensor 40 auf. Bevorzugt umfasst die Sensoreinheit 39 ein Wärmebildsensorarray mit zwei oder mehr Wärmebildsensoren 40. Die einzelnen Wärmebildsensoren 40 umfassen bevorzugt Infrarotsensoren, die zu einer Erfassung von Wärmestrahlung, insbesondere Infrarotstrahlung, ausgebildet sind. In einer alternativen Ausgestaltung der zweiten Erfassungsvorrichtung 37 kann diese auch eine Kamera und/oder weitere, dem Fachmann als sinnvoll erscheinende Sensoreinheiten 39 umfassen.

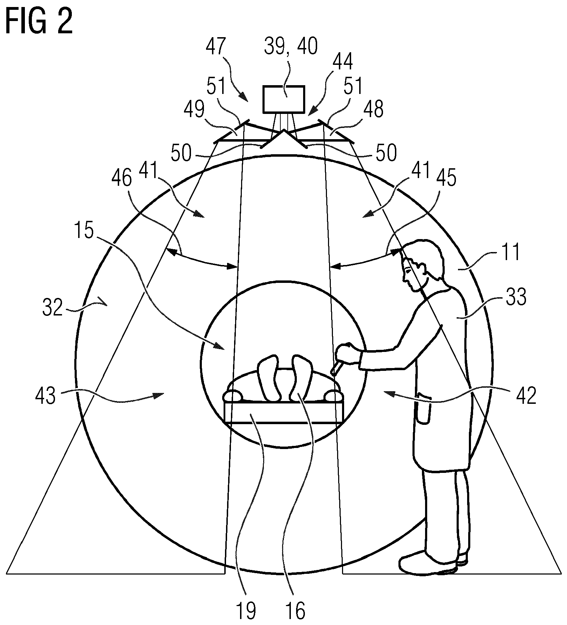

Die Sensoreinheit 39 weist ein Sichtfeld 41 auf, das einen Bereich 42, 43 neben dem Liegentisch 19, auf dem das Objekt, insbesondere der Patient 16, positioniert ist, umfasst. Die zweite Erfassungsvorrichtung 37 weist zudem eine Sichtfeldmanipulationseinheit 44 auf, die dazu ausgebildet ist, einen Bereich des Liegentischs 19 aus dem Sichtfeld 41 der Sensoreinheit 39 auszublenden und/oder zu eliminieren, wie dies in Fig. 2 dargestellt ist. Dabei ist die Sichtfeldmanipulationseinheit 44 zu einer Teilung und/oder Umlenkung des Sichtfelds 41 der Sensoreinheit 39 in zwei Teilsichtfelder 45, 46 ausgebildet. Bevorzugt umfasst ein erstes Teilsichtfeld 45 einen Bereich 42 rechts neben der Patientenlagerungsvorrichtung 17, insbesondere dem Liegentisch 19 der Patientenlagerungsvorrichtung 17. Zudem umfasst dabei ein zweites Teilsichtfeld 46 einen Bereich 43 links neben der Patientenlagerungsvorrichtung 17, insbesondere dem Liegentisch 19 der Patientenlagerungsvorrichtung 17.

Zur Teilung und/oder Umlenkung des Sichtfelds 41 der Sensoreinheit 39 weist die Sichtfeldmanipulationseinheit 44 ein Spiegelsystem 47 mit zwei Erfassungspfaden 48, 49 auf, wobei jeder Erfassungspfad 48, 49 zumindest einen Spiegel 50, 51 zu einer Umlenkung eines Teilsichtfelds 45, 46 umfasst. Im vorliegenden Ausführungsbeispiel weist jeder Erfassungspfad 48, 49 des Spiegelsystems 47 zwei Spiegel 50, 51 auf zu einer Umlenkung eines Teilsichtfelds 45, 46. Dabei wird das Teilsichtfeld 45, 46 der Sensoreinheit 39 jeweils auf den ersten Spiegel 50, von dem ersten Spiegel 50 auf den zweiten Spiegel 51 und von dem zweiten Spiegel 51 auf den Bereich 42, 43 neben dem Liegentisch 19, insbesondere links oder rechts neben dem Liegentisch 19, gelenkt. Vorzugsweise sind die zwei Spiegel 50, 51 eines Erfassungspfades 48, 49 derart angeordnet, dass der Liegentisch 19 außerhalb der Teilsichtfelder 45, 46 angeordnet ist, jedoch die Bereiche 42, 43 neben dem Liegentisch 19 vollständig erfasst werden.

Die zweite Erfassungsvorrichtung 37 weist zudem eine Auswerteeinheit 52 auf, die zu einer Auswertung der erfassten Positionsdaten des medizinischen Bedienpersonals 33 ausgebildet ist (Fig. 1). Die Auswerteeinheit 52 umfasst bevorzugt eine Recheneinheit, insbesondere eine Mikrokontroller, der zu Auswertung der erfassten Positionsdaten des medizinischen Bedienpersonals 33 ausgebildet ist. Zudem umfasst die Auswerteeinheit 52 eine Software, die zu einer Auswertung der mittels der Sensoreinheit 39 erfassten Positionsdaten des medizinischen Bedienpersonals 33 ausgebildet ist. Bevorzugt wird bei der Auswertung der mittels der Sensoreinheit 39 erfassten Positionsdaten eine Position des medizinischen Bedienpersonals 33 bezogen auf die z-Richtung und/oder die Längsrichtung 36 des Liegentischs 19 ermittelt und/oder bestimmt.

Zu einer Datenübertragung einer Position des medizinischen Bedienpersonals 33 weist die zweite Erfassungsvorrichtung 37 eine optische Datenübertragungseinheit 53 auf. Im vorliegenden Ausführungsbeispiel erfolgt die Datenübertragung mittels der optischen Datenübertragungseinheit 53 von der zweiten Erfassungsvorrichtung 37 an die Linearachseneinheit 29. In einer alternativen Ausgestaltung kann die Datenübertragung mittels der optischen Datenübertragungseinheit 53 auch an die Magneteinheit 11 und/oder an die Systemsteuereinheit 23 der Magnetresonanzvorrichtung 10 erfolgen. Zur Datenübertragung weist die optische Datenübertragungseinheit 53 zumindest einen Lichtwellenleiter 54 auf, der die zweite Erfassungsvorrichtung 37 mit der Linearachseneinheit 29 verbindet. Bevorzugt weist hierbei die zweite Erfassungsvorrichtung 37, insbesondere die Auswerteeinheit 52 der zweiten Erfassungsvorrichtung 37, eine entsprechende optische Datenschnittstelle auf.

Die dargestellte medizinische Bildgebungsvorrichtung 56, insbesondere die Magnetresonanzvorrichtung 10, kann selbstverständlich weitere Komponenten umfassen, die medizinische Bildgebungsvorrichtungen 56, insbesondere Magnetresonanzvorrichtungen 10, gewöhnlich aufweisen. Eine allgemeine Funktionsweise einer medizinischen Bildgebungsvorrichtung 56, insbesondere die Magnetresonanzvorrichtung 10, ist zudem dem Fachmann bekannt, so dass auf eine detaillierte Beschreibung der weiteren Komponenten verzichtet wird.

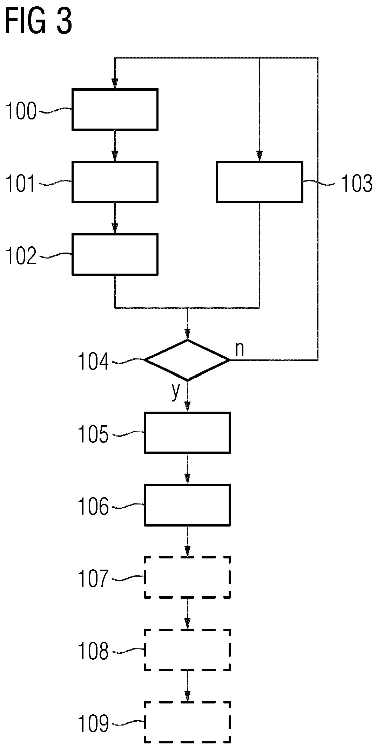

In Fig. 3 ist ein Verfahren zu einem Erfassen einer Position eines zu untersuchenden Bereichs eines Objekts, insbesondere eines Patienten 16, mittels der ersten Erfassungsvorrichtung 27 für eine medizinische Bildgebungsuntersuchung, insbesondere eine Magnetresonanzuntersuchung, dargestellt. Zu einer Steuerung des Verfahrens weist die medizinische Bildgebungsvorrichtung, insbesondere die Magnetresonanzvorrichtung 10, eine Recheneinheit 55 auf, die die einzelnen Verfahrensschritte des Verfahrens zu einem Erfassen einer Position eines zu untersuchenden Bereichs eines Objekts, insbesondere eines Patienten 16, steuert. Die Recheneinheit 55 ist hierbei von der Systemsteuereinheit 23 umfasst. Die Recheneinheit 55 weist hierzu eine Steuersoftware und/oder Computerprogramme auf, die in einem Speicher der Recheneinheit 55 gespeichert sind. Bei einem Ausführen der Steuersoftware und/oder der Computerprogramme durch einen Prozessor der Recheneinheit 55 werden die einzelnen Verfahrensschritte entsprechend angesteuert.

In einem ersten Verfahrensschritt 100 erfolgt ein Vorbereiten des Objekts auf dem Liegentisch 19 der Patientenlagerungsvorrichtung 17. Hierzu befindet sich der Liegentisch 19 vor dem Aufnahmebereich 15 der Magnetresonanzvorrichtung 10 in einer Grundposition. Der erste Verfahrensschritt 100, insbesondere das Vorbereiten des Objekts und/oder des Patienten 16, erfolgt dabei manuell durch das medizinische Bedienpersonal 33.

In einem zweiten Verfahrensschritt 101 erfolgt ein Erfassen von Positionsdaten des medizinischen Bedienpersonals 33 mittels der zweiten Erfassungsvorrichtung 37. Das Erfassen der Positionsdaten des medizinischen Bedienpersonals 33 erfolgt dabei während des manuellen Vorbereitens des Objekts, insbesondere des Patienten 16, auf dem Liegentisch 19. Beim Erfassen der Positionsdaten des medizinischen Bedienpersonals 33 mittels der zweiten Erfassungsvorrichtung 37 wird in einem Sichtfeld 41 der zweiten Erfassungsvorrichtung 37, insbesondere der Sensoreinheit 39 mit den mehreren Wärmebildsensoren 40, ein Bereich des Liegentischs 19 ausgeblendet und/oder eliminiert. Hierzu wird, wie dies bereits zu den Ausführungen zu den Fig. 1 und 2 beschrieben ist, das Sichtfeld 41 der zweiten Erfassungsvorrichtung 37, insbesondere der Sensoreinheit 39, geteilt und/oder umgelenkt. Insbesondere wird hierbei das Sichtfeld 41 der Sensoreinheit 39 in zwei Erfassungspfade 48, 49 mittels der Sichtfeldmanipulationseinheit 53 aufgeteilt, wobei die zwei Erfassungspfade 48, 49 die Bereiche 42, 43 links und rechts neben dem Liegentisch 19 und damit einen möglichen Aufenthaltsort des medizinischen Bedienpersonals 33 erfassen und den Liegentisch 19 aus dem Sichtfeld 41 eliminieren. Insbesondere wird hierbei das Sichtfeld 41 der Sensoreinheit 39 in zwei Teilsichtfelder 45, 46 aufgeteilt.

In einem weiteren, dritten Verfahrensschritt 102 erfolgt ein Nachführen der Projektionsvorrichtung 28 in Längsrichtung 36 des Liegentischs 19 an eine Position des medizinischen Bedienpersonals 33. Dabei erfolgt das Nachführen auf Basis einer anhand der aus den erfassten Positionsdaten ermittelten Positionsinformation des medizinischen Bedienpersonals 33. Dabei werden die erfassten Positionsdaten mittels der Auswerteeinheit 52, insbesondere des Mirkokontrollers, der zweiten Erfassungsvorrichtung 37 ausgewertet und eine Position des medizinischen Bedienpersonals 33 bezüglich der Längsrichtung 36 des Liegentischs 19 bestimmt. Anschließend wird in diesem dritten Verfahrensschritt 102 die ermittelte Positionsinformation des medizinischen Bedienpersonals 33 an die Linearachseneinheit 29 übertragen, insbesondere mittels der optischen Datenübertragungseinheit 53 der zweiten Erfassungsvorrichtung 37. Mittels der Linearachseneinheit 29 wird anschließend die Projektionsvorrichtung 28 an die ermittelte Position des medizinischen Bedienpersonals 33 nachgeführt, insbesondere in Längsrichtung 36 des Liegentischs 19.

Gleichzeitig mit den Verfahrensschritten 100, 101, 102 wird in einem weiteren Verfahrensschritt 103 mittels der ersten Erfassungsvorrichtung 27 ständig der Bereich des Liegentischs 19 erfasst, um ein mögliches Markierelement 34 zu erfassen. Das Markierelement 34 kann dabei einen Finger des medizinischen Bedienpersonals 33 umfassen oder auch einen handgeführten Gegenstand, wie beispielsweise einen Markierstab. Hierzu weist auch die erste Erfassungsvorrichtung 27 eine entsprechende Software und/oder Computerprogramme auf, um das Markierelement 34 in den erfassten Bilddaten, insbesondere Kameradaten, zu erkennen. Das Positionieren des Markierelements 34 erfolgt manuell durch das medizinische Bedienpersonal 33, wobei das Markierelement 34 an den zu untersuchenden Bereich des Objekts, insbesondere des Patienten 16, positioniert wird.

In einem weiteren Verfahrensschritt 104, wird ermittelt, ob das Markierelement 34 von der ersten Erfassungsvorrichtung 27 erfasst und erkannt wurde oder nicht. Sofern kein Markierelement 34 von der ersten Erfassungsvorrichtung 27 erfasst und erkannt wurde, werden die Verfahrensschritte 100, 101, 102, 103 so lange wiederholt, bis der zu untersuchende Bereich des Objekts, insbesondere des Patienten 16, mittels eines Positionierens des Markierelements 34 festgelegt wird.

Sobald das Markierelement 34 von der ersten Erfassungsvorrichtung 27 erfasst und erkannt wurde, erfolgt in einem weiteren Verfahrensschritt 105 ein Erfassen der Position des Markierelements 34 mittels der ersten Erfassungsvorrichtung 27. In diesem Verfahrensschritt 105 werden zudem eine Positionsinformation des Markierelements 34 von der ersten Erfassungsvorrichtung 27 an die Linearachseneinheit 29 übertragen.

In einem weiteren Verfahrensschritt 106 wird zunächst die Projektionsvorrichtung 28 in Längsrichtung 36 des Liegentischs 19 mittels der Linearachseneinheit 29 an die Position des Markierelements 34 gefahren. Dabei wird die Projektionsvorrichtung 28 von der zuletzt eingestellten Position, insbesondere der Position des medizinischen Bedienpersonals 33 bezogen auf die Längsrichtung des Patiententischs 16, an die Position des Markierelement 34 gefahren. Anschließend erfolgt mittels der Projektionsvorrichtung 28 eine Projektion und/oder Anzeige einer Feedback-Markierung an der erfassten Position des zu untersuchenden Bereichs.

In einem weiteren optionalen Verfahrensschritt 107 wird anhand der erfassten Position des zu untersuchenden Bereichs des Objekts, insbesondere des Patienten 16, eine Untersuchungsposition des Liegentischs 19 für die anstehende medizinische Bildgebungsuntersuchung, insbesondere Magnetresonanzuntersuchung, berechnet. In einem weiteren optionalen Verfahrensschritt 108 wird anschließend der Liegentisch 19 zusammen mit dem Objekt, insbesondere mit dem Patienten 16, in diese Untersuchungsposition gefahren für eine Erfassung von medizinischen Bildgebungsdaten, insbesondere von Magnetresonanzdaten, des zu untersuchenden Bereichs. In dieser Untersuchungsposition befindet sich der zu untersuchende Bereich des Objekts, insbesondere des Patienten 16, innerhalb des Aufnahmebereichs 15, insbesondere des Isozentrums, der medizinischen Bildgebungsvorrichtung 56, insbesondere der Magnetresonanzvorrichtung 10. Anschließend wird in einem weiteren optionalen Verfahrensschritt 109 die medizinische Bildgebungsuntersuchung, insbesondere die Magnetresonanzuntersuchung, des zu untersuchenden Bereichs des Objekts, insbesondere des Patienten 16, durchgeführt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizinische Bildgebungsvorrichtung (56) umfassend:
- einen Liegentisch (19), auf dem ein Objekt für eine medizinische Bildgebungsuntersuchung positionierbar ist und der in einen Aufnahmebereich (15) der medizinische Bildgebungsvorrichtung (56) einfahrbar ist,
- eine erste Erfassungsvorrichtung (27), die zu einem Erfassen einer Position eines zu untersuchenden Bereichs des Objekts für eine medizinische Bildgebungsuntersuchung ausgebildet ist,
- eine Projektionsvorrichtung (28), die zu einer Projektion und/oder Anzeige einer Feedback-Markierung an der erfassten Position des zu untersuchenden Bereichs ausgebildet ist,
- einer Linearachseneinheit (29), die eine Antriebseinheit zu einem Bewegen der Projektionsvorrichtung (28) umfasst, wobei die Linearachseneinheit (29) parallel zur Längsrichtung (36) des Liegentischs (19) über dem Liegentisch (19) angeordnet ist,
**gekennzeichnet durch** eine zweite Erfassungsvorrichtung (37), die zu einem Erfassen von Positionsdaten eines medizinischen Bedienpersonals (33) während eines Vorbereitens des Objekts für die medizinische Bildgebungsuntersuchung ausgebildet ist, wobei die Projektionsvorrichtung (28) anhand der erfassten Positionsdaten der zweiten Erfassungsvorrichtung (37) an eine Position des medizinischen Bedienpersonals (33) bezogen auf eine Längsrichtung (36) des Liegentischs (19) an der Linearachseneinheit (29) während der Vorbereitung des Objekts nachgeführt wird.

2. Medizinische Bildgebungsvorrichtung (56) nach Anspruch 1,
**gekennzeichnet durch** eine Magnetresonanzvorrichtung (10).

3. Medizinische Bildgebungsvorrichtung (56) nach einem der vorhergehenden Ansprüche,
**dadurch gekennzeichnet, dass** die zweite Erfassungsvorrichtung (37) eine Sensoreinheit (39) zur Erfassung der Positionsdaten des medizinischen Bedienpersonals (33) umfasst, wobei die Sensoreinheit (39) ein Sichtfeld (41) aufweist, das einen Bereich (42, 43) neben dem Liegentisch (19) umfasst.

4. Medizinische Bildgebungsvorrichtung (56) nach Anspruch 3,
**dadurch gekennzeichnet, dass** die Sensoreinheit (39) zumindest einen Wärmebildsensor (40) umfasst.

5. Medizinische Bildgebungsvorrichtung (56) nach einem der Ansprüche 3 oder 4,
**dadurch gekennzeichnet, dass** die zweite Erfassungsvorrichtung (37) eine Sichtfeldmanipulationseinheit (44) umfasst, die dazu ausgebildet ist, einen Bereich des Liegentischs (19) aus dem Sichtfeld (41) der Sensoreinheit (39) auszublenden.

6. Medizinische Bildgebungsvorrichtung (56) nach Anspruch 5,
**dadurch gekennzeichnet, dass** die Sichtfeldmanipulationseinheit (44) zu einer Teilung und/oder Umlenkung des Sichtfelds (41) der Sensoreinheit (39) in zwei Teilsichtfelder (45, 46) ausgebildet ist.

7. Medizinische Bildgebungsvorrichtung (56) nach einem der Ansprüche 5 oder 6,
**dadurch gekennzeichnet, dass** die Sichtfeldmanipulationseinheit (44) ein Spiegelsystem (47) mit zwei Erfassungspfaden (48, 49) umfasst, wobei jeder Erfassungspfad (48, 49) zumindest einen Spiegel (50, 51) zu einer Umleitung eines Teilsichtfelds (45, 46) aufweist.

8. Medizinische Bildgebungsvorrichtung (56) nach einem der vorhergehenden Ansprüche,
**dadurch gekennzeichnet, dass** die zweite Erfassungsvorrichtung (37) eine Auswerteeinheit (52) aufweist, die zu einer Auswertung der erfassten Positionsdaten ausgebildet ist.

9. Medizinische Bildgebungsvorrichtung (56) nach einem der vorhergehenden Ansprüche,
**dadurch gekennzeichnet, dass** die zweite Erfassungsvorrichtung (37) ein Schirmgehäuse (38) umfasst.

10. Medizinische Bildgebungsvorrichtung (56) nach einem der vorhergehenden Ansprüche,
**dadurch gekennzeichnet, dass** die zweite Erfassungsvorrichtung (37) eine optische Datenübertragungseinheit (53) aufweist zu einem Übertragen einer Positionsinformation des medizinischen Bedienpersonals (33) an die medizinische Bildgebungsvorrichtung (56) und/oder an die Linearachseneinheit (29).

11. Verfahren zu einem Erfassen einer Position eines zu untersuchenden Bereichs eines Objekts mittels einer ersten Erfassungsvorrichtung (27) für eine medizinische Bildgebungsuntersuchung, umfassend die folgenden Verfahrensschritte:
- Vorbereiten des Objekts auf einem in einem Aufnahmebereich (15) einer medizinische Bildgebungsvorrichtung (56) einfahrbaren Liegentisch (19), wobei das Vorbereiten des Objekts manuell durch ein medizinisches Bedienpersonal (33) erfolgt,
- Erfassen von Positionsdaten des medizinischen Bedienpersonals (33) mittels einer zweiten Erfassungsvorrichtung (37) während des Vorbereitens des Objekts,
- Nachführen einer Projektionsvorrichtung (28) in Längsrichtung (36) des Liegentischs (19) an eine Position des medizinischen Bedienpersonals (33), wobei das Nachführen auf Basis einer anhand der erfassten Positionsdaten ermittelten Positionsinformation des medizinischen Bedienpersonals (33) erfolgt,
- Wiederholen der Verfahrensschritte des Positionierens des Objekts, des Erfassens der Positionsdaten des medizinischen Bedienpersonals (33) und des Verfahrensschritts des Nachführens der Projektionsvorrichtung (28) so lange, bis der zu untersuchende Bereich des Objekts mittels eines manuellen Positionierens eines Markierelements (34) festgelegt wird,
- Erfassen der Position des Markierelements (34) mittels der ersten Erfassungsvorrichtung (27), und
- Projektion und/oder Anzeige einer Feedback-Markierung durch eine Projektionsvorrichtung (28) an der erfassten Position des zu untersuchenden Bereichs.

12. Verfahren nach Anspruch 11,
**dadurch gekennzeichnet, dass** zum Erfassen der Positionsdaten des medizinischen Bedienpersonals (33) in dem Sichtfeld (41) der zweiten Erfassungsvorrichtung (37) ein Bereich des Liegentischs (19) ausgeblendet wird.

13. Verfahren nach einem der Ansprüche 11 bis 12,
**dadurch gekennzeichnet, dass** zum Erfassen der Positionsdaten des medizinischen Bedienpersonals (33) ein Sichtfeld (41) der zweiten Erfassungsvorrichtung (37) geteilt und/oder umgelenkt wird.

14. Verfahren nach einem der Ansprüche 11 bis 13,
**dadurch gekennzeichnet, dass** mittels der zweiten Erfassungsvorrichtung (37) die erfassten Positionsdaten ausgewertet werden und eine ermittelte Positionsinformation des medizinischen Bedienpersonals (33) mittels einer optischen Datenübertragungseinheit an eine Linearachseneinheit (29) zur Positionierung der Projektionsvorrichtung (28) übertragen werden.

15. Verfahren nach einem der Ansprüche 11 bis 14,
**dadurch gekennzeichnet, dass** anhand der erfassten Position des zu untersuchenden Bereichs des Objekts eine Untersuchungsposition des Liegentischs (19) berechnet wird.

16. Verfahren nach Anspruch 15,
**dadurch gekennzeichnet, dass** der Liegentisch (19) automatisch in die Untersuchungsposition gefahren wird für eine Erfassung von medizinischen Bildgebungsdaten des zu untersuchenden Bereichs des Objekts.

17. Computerprogrammprodukt, welches ein Programm umfasst und direkt in einen Speicher einer programmierbaren Steuerungseinheit ladbar ist, mit Programmmitteln, um ein Verfahren zu einem Erfassen einer Position eines zu untersuchenden Bereichs eines Objekts mittels einer ersten Erfassungsvorrichtung (27) für eine medizinische Bildgebungsuntersuchung nach einem der Ansprüche 11 bis 16 auszuführen, wenn das Programm in der Steuerungseinheit ausgeführt wird.

## Claims

1. Medical imaging apparatus (56) comprising:
- a couch (19) on which an object can be positioned for a medical imaging examination and which can be moved into a recording region (15) of the medical imaging apparatus (56),
- a first acquisition apparatus (27) which is embodied for acquiring a position of a region to be examined of the object for a medical imaging examination,
- a projection apparatus (28) which is embodied for a projection and/or display of a feedback marking at the acquired position of the region to be examined,
- a linear axis unit (29) which comprises a drive unit for moving the projection apparatus (28), wherein the linear axis unit (29) is arranged above the couch (19) parallel to the longitudinal direction (36) of the couch (19),
**characterised by** a second acquisition apparatus (37) which is embodied to acquire position data of a medical operator (33) during preparation of the object for the medical imaging examination, wherein the projection apparatus (28), using the acquired position data of the second acquisition apparatus (37), is tracked to a position of the medical operator (33) relative to a longitudinal direction (36) of the couch (19) on the linear axis unit (29) during preparation of the object.

2. Medical imaging apparatus (56) according to claim 1, **characterised by** a magnetic resonance apparatus (10).

3. Medical imaging apparatus (56) according to one of the preceding claims,
**characterised in that** the second acquisition apparatus (37) comprises a sensor unit (39) for acquiring the position data of the medical operator (33), wherein the sensor unit (39) has a field of view (41) which comprises a region (42, 43) next to the couch (19).

4. Medical imaging apparatus (56) according to claim 3, **characterised in that** the sensor unit (39) comprises at least one thermal imaging sensor (40).

5. Medical imaging apparatus (56) according to one of claims 3 or 4,
**characterised in that** the second acquisition apparatus (37) comprises a field of view manipulation unit (44) which is embodied to fade out a region of the couch (19) from the field of view (41) of the sensor unit (39).

6. Medical imaging apparatus (56) according to claim 5, **characterised in that** the field of view manipulation unit (44) is embodied for dividing and/or deflecting the field of view (41) of the sensor unit (39) into two partial fields of view (45, 46).

7. Medical imaging apparatus (56) according to one of claims 5 or 6,
**characterised in that** the field of view manipulation unit (44) comprises a mirror system (47) with two acquisition paths (48, 49), wherein each acquisition path (48, 49) has at least one mirror (50, 51) for diverting a partial field of view (45, 46).

8. Medical imaging apparatus (56) according to one of the preceding claims,
**characterised in that** the second acquisition apparatus (37) has an evaluation unit (52) which is embodied for an evaluation of the acquired position data.

9. Medical imaging apparatus (56) according to one of the preceding claims,
**characterised in that** the second acquisition apparatus (37) comprises a shielding housing (38).

10. Medical imaging apparatus (56) according to one of the preceding claims,
**characterised in that** the second acquisition apparatus (37) has an optical data transfer unit (53) for transferring an item of position information of the medical operator (33) to the medical imaging apparatus (56) and/or to the linear axis unit (29).

11. Method for acquiring a position of a region to be examined of an object by means of a first acquisition apparatus (27) for a medical imaging examination, comprising the following method steps:
- preparing the object on a couch (19) which can be moved in a recording region (15) of a medical imaging apparatus (56), wherein the object is prepared manually by a medical operator (33),
- acquiring position data of the medical operator (33) by means of a second acquisition apparatus (37) during preparation of the object,
- tracking a projection apparatus (28) in the longitudinal direction (36) of the couch (19) to a position of the medical operator (33), wherein the tracking takes place on the basis of an item of position information of the medical operator (33) ascertained using the acquired position data,
- repeating the method steps of positioning the object, of acquiring the position data of the medical operator (33) and the method step of tracking the projection apparatus (28) until the region to be examined of the object is defined by means of manual positioning of a marker element (34),
- acquiring the position of the marker element (34) by means of the first acquisition apparatus (27), and
- projection and/or display of a feedback marking by a projection apparatus (28) at the acquired position of the region to be examined.

12. Method according to claim 11,
**characterised in that** a region of the couch (19) is faded out for acquiring the position data of the medical operator (33) in the field of view (41) of the second acquisition apparatus (37).

13. Method according to one of claims 11 to 12, **characterised in that** a field of view (41) of the second acquisition apparatus (37) is divided and/or deflected for acquiring the position data of the medical operator (33).

14. Method according to one of claims 11 to 13, **characterised in that** the acquired position data is evaluated by means of the second acquisition apparatus (37) and an ascertained item of position information of the medical operator (33) is transferred to a linear axis unit (29) by means of an optical data transfer unit for positioning the projection apparatus (28).

15. Method according to one of claims 11 to 14, **characterised in that** an examination position of the couch (19) is calculated using the acquired position of the region to be examined of the object.

16. Method according to claim 15,
**characterised in that** the couch (19) is automatically moved into the examination position for acquisition of medical imaging data of the region of the object to be examined.

17. Computer program product which comprises a program and can be loaded directly into a memory of a programmable control unit, with program means in order to execute a method for acquiring a position of a region to be examined of an object by means of a first acquisition apparatus (27) for a medical imaging examination according to one of claims 11 to 16 when the program is executed in the control unit.

## Revendications

1. Dispositif (56) d'imagerie médicale comprenant :
- une couchette (19), sur laquelle un objet pour un examen d'imagerie médicale peut être mis en position et qui peut être entrée dans une zone (15) d'enregistrement du dispositif (56) d'imagerie médicale,
- un premier dispositif (27) de détection, qui est constitué pour la détection d'une position d'une zone à examiner de l'objet pour un examen d'imagerie médicale,
- un dispositif (28) de projection, qui est constitué pour une projection et/ou un affichage d'un repérage de feedback à la position détectée de la zone à examiner,
- une unité (29) à axe linéaire, qui comprend une unité d'entraînement pour le déplacement du dispositif (28) de projection, dans laquelle l'unité (29) à axe linéaire est disposée parallèlement à la direction (36) longitudinale de la couchette (19) au-dessus de la couchette (19),
**caractérisé par** un deuxième dispositif (37) de détection, qui est constitué pour la détection de données de position d'une personne (33) de service médical pendant une préparation de l'objet pour l'examen d'imagerie médicale, dans lequel le dispositif (28) de projection est, pendant le prétraitement de l'objet, suivi, à l'aide des données de position détectées du deuxième dispositif (37) de détection, en une position du personnel (33) de service médical rapportée à une direction (36) longitudinale de la couchette (19) sur l'unité (29) à axe linéaire.

2. Dispositif (56) d'imagerie médicale suivant la revendication 1,
**caractérisé par** un dispositif (10) à résonnance magnétique.

3. Dispositif (56) d'imagerie médicale suivant l'une des revendications précédentes,
**caractérisé en ce que** le deuxième dispositif (37) de détection comprend une unité (39) de capteur pour la détection des données de position de la personne (33) de service médical, dans lequel l'unité (39) de capteur a un champ (41) de vue, qui comprend une zone (42, 43) à côté de la couchette (19).

4. Dispositif (56) d'imagerie médicale suivant la revendication 3,
**caractérisé en ce que** l'unité (39) de capteur comprend au moins un capteur (40) d'image thermique.

5. Dispositif (56) d'imagerie médicale suivant l'une des revendications 3 ou 4,
**caractérisé en ce que** le deuxième dispositif (37) de détection comprend une unité (44) de manipulation de champ de vue, qui est constituée pour supprimer une zone de la couchette (19) du champ (41) de vue de l'unité (39) de capteur.

6. Dispositif (56) d'imagerie médicale suivant la revendication 5,
**caractérisé en ce que** l'unité (44) de manipulation de champ de vue est constituée pour la séparation et/ou l'inversion du champ (41) de vue de l'unité (39) de capteur en deux champs (45, 46) de vue partiels.

7. Dispositif (56) d'imagerie médicale suivant l'une des revendications 5 ou 6,
**caractérisé en ce que** l'unité (44) de manipulation de champ de vue comprend un système (47) de miroir ayant deux chemins (48, 49) de détection, dans lequel chaque chemin (48, 49) de détection a au moins un miroir (50, 51) de déviation d'un champ (45, 46) de vue partiel.

8. Dispositif (56) d'imagerie médicale suivant l'une des revendications précédentes,
**caractérisé en ce que** le deuxième dispositif (37) de détection a une unité (52) d'évaluation, qui est constituée pour l'évaluation des données de position détectées.

9. Dispositif (56) d'imagerie médicale suivant l'une des revendications précédentes,
**caractérisé en ce que** le deuxième dispositif (37) de détection comprend un boîtier (38) de protection.

10. Dispositif (56) d'imagerie médicale suivant l'une des revendications précédentes,
**caractérisé en ce que** le deuxième dispositif (37) de détection a une unité (53) optique de transmission de données pour la transmission d'une information de position du personnel (33) de service médical au dispositif (56) d'imagerie médicale et/ou à l'unité (29) à axe linéaire.

11. Procédé de détection de la position d'une partie à examiner d'un objet au moyen d'un premier dispositif (27) de détection pour un examen d'imagerie médicale, comprenant les stades de procédé suivants :
- préparation de l'objet sur une couchette (19) pouvant être rentrée dans une zone (15) d'enregistrement d'un dispositif (56) d'imagerie médicale, dans lequel la préparation de l'objet s'effectue manuellement par un personnel (33) de service médical,
- détection de données de position du personnel (33) de service médical au moyen d'un deuxième dispositif (37) de détection pendant la préparation de l'objet,
- suivi d'un dispositif (28) de projection dans la direction (36) longitudinale de la couchette (19) en une position du personnel (33) de service médical, dans lequel le suivi s'effectue sur la base d'une information de position, déterminée à l'aide des données de position détectées, du personnel (33) de service médical,
- répétition des stades de procédé de la mise en position de l'objet, de la détection des données de position du personnel (33) de service médical et du stade de procédé du suivi du dispositif (28) de projection, jusqu'à ce que la zone à examiner de l'objet soit déterminée au moyen d'une mise en position manuelle d'un élément (34) de marquage,
- détection de l'élément (34) de marquage au moyen du premier dispositif (27) de détection, et
- projection et/ou affichage d'un marquage de feedback par un dispositif (28) de projection en la position détectée de la zone à examiner.

12. Procédé suivant la revendication 11,
**caractérisé en ce que**, pour la détection des données de position du personnel (33) de service médical, on supprime, dans le champ (41) de vue du deuxième dispositif (37) de détection, une zone de la couchette (19).

13. Procédé suivant l'une des revendications 11 à 12, **caractérisé en ce que**, pour la détection des données de position du personnel (33) de service médical, on subdivise et/ou on inverse un champ (41) de vue du deuxième dispositif (37) de détection.

14. Procédé suivant l'une des revendications 11 à 13, **caractérisé en ce que**, au moyen du deuxième dispositif (37) de détection, on évalue les données de position détectées et on transmet, pour la mise en position du dispositif (28) de projection, une information de position déterminée du personnel (33) de service médical au moyen d'une unité optique de transmission de données à une unité (29) à axe linéaire.

15. Procédé suivant l'une des revendications 11 à 14, **caractérisé en ce que**, à l'aide de la position détectée de la zone à examiner de l'objet, on calcule une position d'examen de la couchette (19).

16. Procédé suivant la revendication 15,
**caractérisé en ce que** l'on met automatiquement la couchette (19) dans la position d'examen pour une détection de données d'imagerie médicale de la zone à examiner de l'objet.

17. Produit de programme d'ordinateur, qui comprend un programme et qui peut être chargé directement dans la mémoire d'une unité de commande programmable, comprenant des moyens de programme, pour exécuter un procédé de détection de la position d'une zone à examiner d'un objet au moyen d'un premier dispositif (27) de détection pour un examen d'imagerie médicale suivant l'une des revendications 11 à 16, lorsque le programme est exécuté dans l'unité de commande.
